# EUROPÄISCHE PATENTANMELDUNG

(11) **EP 1 106 202 A2**
(43) Veröffentlichungstag der Anmeldung: **13.06.2001**
(21) Anmeldenummer: 00250412.4
(22) Anmeldetag: 30.11.2000
(51) Int. Cl.: A61N 1/05

(54) **Elektrode zu intravaskuläran Stimulation, Kardioversion und/oder Defibrillation**

(30) Priorität: 30.11.1999 DE 19957662
(71) Anmelder: BIOTRONIK Mess- und Therapiegeräte GmbH & Co Ingenieurbüro Berlin, 12359 Berlin (DE)
(72) Erfinder: Schauerte, Patrick, 52134 Herzogenrath (DE)
(74) Vertreter: Eisenführ, Speiser & Partner

(57) **Zusammenfassung**

Elektrode zurintravaskulären Nervenstimulation, Kardioversion bzw.Defibrillation in Form einer Stimulationssonde, die in arteriellen/venösen Gefäßen des Körpers fixierbar ist und über die elektrische oder magnetische Impulse sowie Defibrillations-/Kardioversionsschocks abgebbar sind, welche mit einer Zuleitung versehen ist, wobei eine in axialer Richtung an die Zuleitung anschließende metallische und elektrisch leitfähige, tubuläre, einen Expansionskörper bildende Drahteinheit vorgesehen ist, die in dem entsprechenden Gefäß entfaltbar ist und sich unter Expansion von innen an die Gefäßwand anlegt.

## Beschreibung

Die Erfindung betrifft eine Elektrode zur intravaskulären Stimulation, Kardioversion und/oder Defibrillation in Form einer Stimulationssonde, die in arteriellen/venösen Gefäßen des Körpers fixiert werden kann und über die elektrische oder magnetische Impulse sowie Defibrillations-/Kardioversionsschocks abgeben werden können.

Die elektrische Stimulation von biologischen Geweben ist ein weitverbreitetes therapeutisches Prinzip. So wird eine atriale und/oder ventrikuläre, myokardiale, elektrische Stimulation des Herzens bei bradykarden (langsamen) und tachykarden (schnellen) Herzrhythmusstörungen eingesetzt (Herzschrittmacher bzw. atriale/ventrikuläre Kardioverter-/Defibrillatoren). Auch die elektrische Stimulation des Magen-/Darmtraktes sowie der Harnblase wird bei Motilitätsstörungen des Magen/Darmtraktes und der Harnblase sowie bei operativ geschaffenen Ersatzmägen/ Ersatzdärmen/Ersatzblasen angewandt. Die Elektrostimulation von Nerven kann therapeutisch in der Schmerztherapie eingesetzt werden (sogenannte TENS Geräte) und zur Therapie von Krampfleiden (Epilepsien) benutzt werden. Auch die elektrische Stimulation des kardialen autonomen Nervensystems zur Kontrolle der Herzfrequenz bei tachykarden supraventrikulären Rhythmusstörungen ist bekannt.

Um diese biologischen Gewebe elektrisch stimulieren zu können, werden in der Regel Stimulationselektroden operativ auf oder in dem betreffenden Gewebe verankert. Insbesondere im Rahmen der autonomen Nervenstimulation ist eine operative Fixierung von Stimulationselektroden auf den betreffenden Nerven schwierig, da diese Nerven häufig entlang von Blutgefäßen ziehen, die bei der Elektrodenplazierung verletzt werden können, und es darüber hinaus postoperativ leicht zur Bildung von Narbengewebe um die Nervenstimulationselektrode herum kommt. Letzteres kann zu einer Erhöhung der Stimulationsreizschwelle bis zum Stimulationsverlust führen. Innerhalb der Blutgefäße angeordnete Elektroden, die im Blutsystem schwimmen, eignen sich ebenfalls nicht zur Nervenstimulation.

Im Falle einer Herzstimulation werden hierzu Sonden, die über Blutgefäße in das Herz vorgeschoben werden, im Herzmuskelgewebe verankert. Überwiegend werden diese Sonden transvenös nach Punktion/Inzision der vena cephalica/vena subclavia über die obere Hohlvene in den rechten Vorhof oder in die rechte Hauptkammer vorgeschoben und dort verankert. Seit kurzem wird auch bei speziellen klinischen Fragestellungen eine elektrische Stimulation des linken Vorhofes (z.B. zur Prävention von Vorhofflimmern) oder des linken Ventrikels (bei Herzinsuffizienz) durchgeführt. Die Stimulationselektroden werden hierzu entweder epikardial oder transvenös über den Koronarvenensinus im Bereich des linken Vorhofs/Ventrikels positioniert. Eine Fixierung von Stimulationselektroden im Koronarsinus ist technisch anspruchsvoll und es kann zu einer partiellen Okklusion der Koronarsinusäste durch die Stimulationssonde kommen.

Aus der US-Patentschrift Nr. 5 954 761 ist eine Stimulationselektrode bekannt, welche eine als Stent ausgebildete Fixationseinheit aufweist. Ein Leiter erstreckt sich dabei im Inneren des Stents.

Nachteilig ist dabei, daß der Querschnitt des sich im Innern des Stents erstreckende Teil des Leiters den für den Blutfluß zur Verfügung stehenden Bereich vermindert.

Hiervon ausgehend liegt der Erfindung die Aufgabe zugrunde, eine Elektrode, insbesondere Nervenstimulationssonde, zu schaffen, die in einem Blutgefäß verankert werden kann, ohne zu einer wesentlichen Verminderung des Blutflusses in diesem Gefäß zu führen.

Gemäß der Erfindung besteht die Elektrode aus einem elektrisch leitenden metallenen, tubulären Drahtgebilde, das sich - entsprechend einem Stent - im entsprechenden Gefäß entfaltet wird und sich von innen an die Gefäßwand anlegt. Das elektrisch leitende Drahtgebilde schließt sich an den Leiter der elektrischen Zuleitung in axialer Richtung an. Innerhalb des Drahtgebildes ist damit keine weitere Leitung erforderlich, welche den für den Blutfluß zur Verfügung stehenden Teil des Gefäßes verringern könnte. Besonders vorteilhaft bei der Erfindung ist dabei weiterhin der Umstand, daß auch ein zur Expansion des Drahtgebildes möglicherweise innerhalb desselben vorgesehener Ballon nicht von einer dort verlaufenden Zuleitung behindert wird.

Das elektrisch leitende Drahtgebilde kann dabei als Spule oder Geflecht oder auch als Aussparungen aufweisender expandierbarer zylindrischer Körper ausgebildet sein.

Die Entfaltung des einen Expansionskörper bildenden Drahtgebildes kann aktiv mittels eines pneumatisch oder durch Flüssigkeit aufblasbaren Ballons, der sich im nicht entfalteten Drahtgebilde befindet, erfolgen.

Alternativ kann auch eine passive Entfaltung (Selbstexpansion) des Drahtgebildes nach Entfernung einer Kompressionshülse erfolgen.

Das metallische, elektrisch leitende Material ist elastisch oder plastisch verformbar - je nachdem, ob es sich um einen durch einen Ballon aufweitbaren oder einen selbstexpandierenden Stent handelt.

Die gesamte Oberfläche des Drahtgebildes oder ein oder mehrere elektrisch voneinander isolierte Teile des Drahtgebildes können als Stimulationspol (unipolar/bipolar/multipolar) benutzt werden. Insbesondere können die Elektroden länglich, dem Verlauf eines Nervs entsprechend ausgebildet sein (siehe DE 197 58 114 A1).

Das Drahtgebilde oder elektrisch isolierte Teile des Drahtgebildes werden mit elektrisch leitfähigen Kabeln mit einer Stimulationseinheit verbunden. Es kann unipolar/bipolar/multipolar über die gesamte Drahtgebildeoberfläche oder elektrisch voneinander isolierte Teile des Drahtgebildes stimuliert werden. Auch eine bipolare Stimulation zwischen dem Drahtgebilde/Teilen des Drahtgebildes und eine in der Nähe des Drahtgebildes implantierte weitere herkömmliche Stimulationssonde oder eine weitere Drahtgebildessonde ist möglich. Wenn mehrere in axialer Richtung aufeinanderfolgende Teile oder des Drahtgebildes elektrisch mit einer Zuleitung zu verbinden sind, so verläuft die Zuleitung im Bereich des Drahtgebildes nicht innerhalb derselben, sondern ist (isoliert) in das Drahtgebilde eingeflochten oder bildet in sonstiger Weise einen selbständig innerhalb der Zylinderform in Längsrichtung verlaufenden, elektrisch isolierten Teil des Drahtgebildes.

Eine andere bevorzugte Ausführungsform der erfindungsgemäßen Elektrode sieht die Verwendung als implantierbare Helmholtzspule vor, über die ein magnetisches Wechselfeld zur Nervenstimulation appliziert werden kann.

Eine weitere Version der einen Expansionskörper bildende Drahteinheit besteht aus einem leitfähigen Draht, der zirkulär im Sinne einer Spule gewickelt ist und aus einer festgelegten Anzahl von Windungen besteht. Das Drahtgebilde kann aus einer oder mehreren elektrisch voneinander isolierten Spulen bestehen. Jede dieser Spulen kann elektrisch mit einem Kabel verbunden sein, das Anschluß an eine elektrische Stimulationseinheit besitzt.

Zur Vermeidung von Restenosen kann auch vorgesehen sein, daß die Elektrode von außen induktiv beheizt wird.

Der Spulenstent kann aber auch in Verbindung mit einer in der Nähe implantierten oder externen, außen auf die Körperoberfläche aufgelegten Induktionseinheit, die keine direkte elektrische Verbindung mit dem Drahtgebilde besitzt, als Elektrode benutzt werden. Diese Induktionseinheit erzeugt induktiv über ein magnetisches oder elektrisches Wechselfeld im Spulenstent ein Spannungsfeld oder Magnetfeld, das zur Stimulation von biologischen Geweben wie Nerven oder Muskulatur genutzt werden kann.

Der Diameter und die Länge der einen Expansionskörper bildende Drahteinheit richtet sich nach dem Durchmesser, der Länge und der Krümmung des Blutgefäßes, in das die einen Expansionskörper bildende Drahteinheit implantiert werden soll. Der Durchmesser des entfalteten Drahtgebilde kann über die gesamte Drahtgebildelänge konstant sein oder variieren. So kann z.B. für die Implantation der einen Expansionskörper bildende Drahteinheit in den proximalen Koronarsinus ein konischer Drahtgebilde benutzt werden. Dies ermöglicht einen durchgehenden Wandkontakt der einen Expansionskörper bildende Drahteinheit im Bereich der sich trichterförmig verengenden Koronarsinusmündung. Die Länge der einen Expansionskörper bildende Drahteinheit kann wenige Millimeter betragen (Ringform) oder mehrere Zentimeter ausmachen. Die Oberfläche der einen Expansionskörper bildende Drahteinheit kann ferner mit Medikamenten beschichtet sein, die eine Schädigung des Gefäßes, in dem die einen Expansionskörper bildende Drahteinheit implantiert wird, vermindern soll (z.B. Kortikosteroidbeschichtung).

Die Plazierung der einen Expansionskörper bildende Drahteinheit kann transvaskulär mit oder ohne Röntgendurchleuchtung erfolgen. Hierzu wird der Elektrodenstent inklusive des Elektrodenschaftes, der die elektrische Zuleitung zur einen Expansionskörper bildende Drahteinheit inkorporiert, über einen Führungsdraht in das entsprechende Zielgefäß vorgeschoben. Der Führungsdraht wird zuvor unter Röntgendurchleuchtung oder echokardiographischer Kontrolle im Gefäß positioniert. Die einen Expansionskörper bildende Drahteinheit inklusive des Elektrodenschaftes weist ein zentrales oder exzentrisches Lumen auf, so daß die einen Expansionskörper bildende Drahteinheit über den Führungsdraht in das Gefäß vorgeschoben werden kann. Alternativ kann der einen Expansionskörper bildende Drahteinheit aber auch kein Lumen aufweisen. In diesem Fall wird nur der Drahtgebilde über einen zuvor im Gefäß plazierten Führungsdraht vorgeschoben und der Elektrodenschaft gleitet entlang, jedoch nicht über den Führungsdraht.

Die Aufweitung der einen Expansionskörper bildenden Drahteinheit im Gefäßerfolgt durch einen im Drahtgebilde plazierten, inflatierbaren Ballon. Die Inflation kann pneumatisch oder durch Flüssigkeit erfolgen. Die Ballon kann über einen im Lumen des einen Expansionskörper bildende Drahteinheitnschaftes befindlichen Führungsdraht oder einen außerhalb des Elektrodenschaftes jedoch durch den Drahtgebilde verlaufenden Führungsdraht erfolgen.

Bei der Pulserzeugungseinrichtung handelt es sich um einen implantierbaren Spannungs/Magnetfeldgenerator, der in der Lage ist, elektrische/magnetische Stimulationsimpulse zu erzeugen. Die Impulsdauer kann zwischen 0 bis 20 ms liegen (typischerweise 0.05 bis 5 ms) und die Stimulationsfrequenz zwischen 0 bis 1000 Hz (typischerweise 10 bis 100 Hz zur Nervenstimulation bzw. 0.5 bis 3 Hz zur Myokardstimulation). Die Impulsform kann monophasisch, biphasisch oder triphasisch sein. Eine andere Variante der Pulserzeugungseinrichtung ist in der Lage, ein wechselndes Spannungs-/Magnetfeld zu erzeugen, das in der Drahtgebildespule ein Magnet/Spannungsfeld induziert. Eine solche Stimulationseinheit kann in der Nähe der Drahtgebildes implantiert werden, ohne eine direkte elektrische Verbindung zur Drahtspule zu besitzen. Alternativ kann aber auch eine externe, außen auf die Körperoberfläche auflegbare Stimulationseinheit verwandt zur Erzeugung eines Spannungs-/Magnetfeldes verwandt werden. Eine weitere Variante der Impulserzeugungseinheit ist in der Lage, Hochspannungsimpulse (Defibrillations-/Kardioversionsimpulse) über die einen Expansionskörper bildende Drahteinheit abzugeben (Impulsspannung zwischen 50 und 1000 V, Impulsdauer 0.5 bis 30 ms, Impulsform mono-/bi-/triphasisch).

Die Stimulationseinheit besteht ferner aus einer Erfassungseinheit, die mit einer oder mehreren Meßsonden verbunden ist, die biologische Meßgrößen, wie Herzfrequenz, Blutdruck, Sauerstoffpartialdruck, Repolatisationszeiten und Veränderungen der Erregungsrückbildung des Herzens) erfassen. Eine auf die Erfassungsgrößen ansprechende Starteinheit setzt die Impulserzeugungseinheit in Betrieb, sobald die Meßgröße einen bestimmten programmierten Grenzwert unter-/überschreitet.

Das Wesen der beschriebenen einen Expansionskörper bildende Drahteinheit läßt unterschiedliche Anwendungen zu.
- Stimulation parasympathischer autonomer Nervenfasern zur Senkung der Herzvorhof- und Hauptkammerfrequenz bei tachykarden Herzrhythmusstörungen. Die Implantation der einen Expansionskörper bildende Drahteinheit kann zu diesem Zweck in der arteria/vena jugularis interna oder externa, der oberen Hohlvene, dem proximalen Koronarsinus oder der unteren Hohlvene an der Grenze zum rechten Vorhof erfolgen.
- Stimulation autonomer Nervenfasern zur Verbesserung der Koronararteriendurchblutung. Die Implantation der einen Expansionskörper bildende Drahteinheit kann zu diesem Zweck in der arteria/vena jugularis interna oder externa sowie im Koronarsinus erfolgen.
- Stimulation sympathischer autonomer Nervenfasern zur Behandlung einer arteriellen Hypotonie und Herzpumpenschwäche bei akuter und chronischer Herzinsuffizienz. Die Implantation der einen Expansionskörper bildende Drahteinheit kann zu diesem Zweck in der arteria/vena subclavia, den Pulmonalvenen oder der Aorta erfolgen.
- Stimulation sympathischer autonomer Nervenfasern zur Behandlung einer arteriellen Hypotonie und Bradykardie bei neurokardiogenen Synkopen. Die Implantation der einen Expansionskörper bildende Drahteinheit kann zu diesem Zweck in der arteria/vena subclavia, den Pulmonalvenen oder der Aorta erfolgen.
- Stimulation parasympathischer autonomer Nervenfasern, zur Behandlung tachykarder ventrikulärer Rhythmusstörungen. Die Implantation der einen Expansionskörper bildende Drahteinheit kann zu diesem Zweck im Koronarsinus oder der Pulmonalarterie erfolgen.
- Stimulation parasympatischer Nerven, die die Vorhöfe innervieren zur Verhinderung eines atrialen Remodelling Prozesses. Die Implantation der einen Expansionskörper bildende Drahteinheit kann zu diesem Zweck in der arteria/vena jugularis interna oder externa, der oberen Hohlvene oder der rechten Pulmonalarterie erfolgen.
- Stimulation parasympathischer Nerven, die die Vorhöfe/Ventrikel innervieren Senkung deratrialen/ventrikulären Defibrillationsschwelle. Die Implantation der einen Expansionskörper bildende Drahteinheit kann zu diesem Zweck in der arteria/vena jugularis interna oder externa, der oberen Hohlvene oder der rechten Pulmonalarterie erfolgen.
- Stimulation autonomer parasympathischer Nervenfasern zur Behandlung von zerebralen Krampfleiden (Epilepsien). Die Implantation der einen Expansionskörper bildende Drahteinheit kann zu diesem Zweck in der arteria/vena jugularis interna oder externa erfolgen.
- Stimulation der Karotissinusnerven zur Behandlung von angina pectoris Beschwerden. Die Implantation der einen Expansionskörper bildende Drahteinheit kann zu diesem Zweck in der arteria/vena jugularis interna oder externa erfolgen.
- Stimulation autonomer Nerven, die die Magen-/Darm- und Blasenmotilität regulieren sowie die männliche Erektion steuern. Die Implantation der einen Expansionskörper bildende Drahteinheit kann zu diesem Zweck in der vena cava inferior und ihren Zuflüssen, der aorta abdominalis sowie ihrer Abgänge (z.B. aa. mesentericae) oder den arteriellen und venösen Ilikalgefäßen erfolgen.
- Hochfrequente, unterschwellige elektrische Stimulation des ventrikulären Myokards zur Förderung der Angiogenese nach Herzinfarkten oder myokardialen Durchblutungsstörungen. Eine Implantation von Drahtgebildeelektroden kann hierzu in den Koronararterien oder dem Koronarsinus und seinen Zuflüssen erfolgen.

Nachfolgend wird die Erfindung anhand bevorzugter Ausführungsbeispiele in Verbindung mit der Zeichnung näher beschrieben. Dabei zeigen:
- Fig. 1: eine erste Ausführungsform mit einer Drahteinheit, die als zylinderförmiges Geflecht ausgebildet ist,
- Fig. 2: eine Elektrode mit Ballon zum Entfalten,
- Fig. 3: eine Fig. 2 entsprechend der Ausführungsform mit nur einer elektrischen Verbindung und einem Führungsdraht,
- Fig. 4: eine in Längsrichtung elektrisch geteilte bipolare Ausführungsform,
- Fig. 5: eine in axialer Richtung geteilte bipolare Ausführungsform,
- Fig. 6: eine Ausführungsform für die obere Hohlvene,
- Fig. 7: eine Ausführungsform für den Koronarvenensinus,
- Fig. 8: eine konisch aufgeweitete Ausführungsform für den Koronarsinus ostium,
- Fig. 9: eine Ausführungsform als schmaler Ring,
- Fig. 10: eine spulenförmige Ausführungsform zur induktiven Erregung durch ein externes Wechselfeld,
- Fig. 11: eine spulenförmige Ausführungsform zur Erregung über eine interne Spule an einem Führungsdraht und
- Fig. 12: eine Ausführungsform mit angeschlossener Stimulations-Steuereinheit.

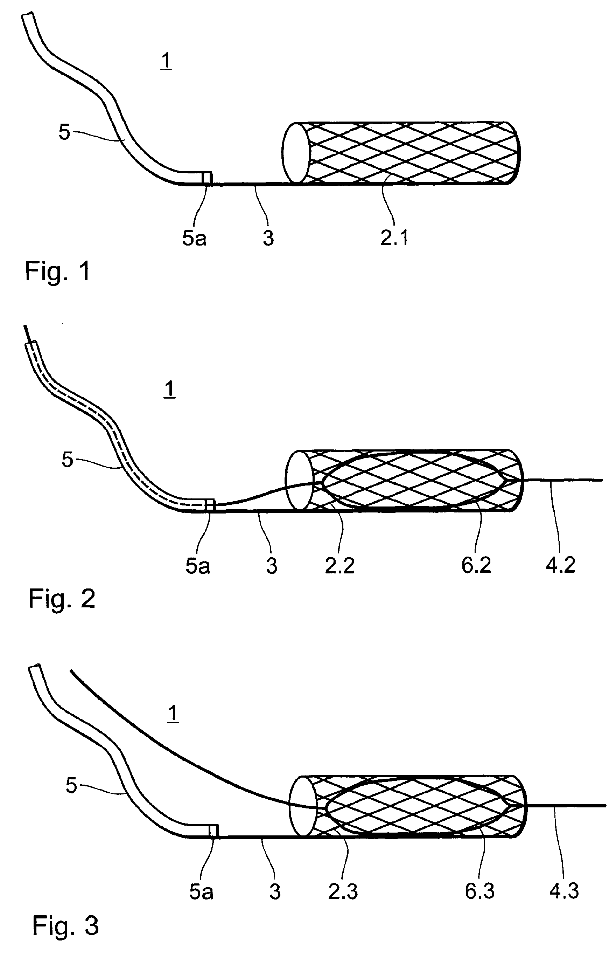

Bei der in Fig. 1 dargestellten Ausführungsform umfaßt die erfindungsgemäße Elekrode 1 eine zylinderförmige Drahteinheit 2.1, die eine bipolare Referenzelektrode bildet. Die Drahteinheit 2.1 besteht aus einem elektrisch leitenden, metallischen Drahtgebilde, welches expandierbar - im Falle einer elastischen Ausgestaltung - selbstexpandierbar ist. Die flexible Elektrodenzuleitung (Sonde) 5 ist mit einem Ring 5a abgeschlossen, welche eine bipolare Referenzelektrode bildet. Zwischen dem Ende der (elektrisch isolierten) Zuleitung und der Drahteinheit 2.1 ist eine elektrische Verbindung 3 vorgesehen. Es ist ersichtlich, daß die Drahteinheit 2 und die Zuleitungen in axialer Richtung aufeinanderfolgend angeordnet sind. Das Innere der zylindrischen Drahteinheit ist völlig frei, so daß der Blutfluß in dem Gefäß nicht behindert wird.

Bei der Ausführungsform gemäß Fig. 2 ist veranschaulicht, wie die - hier plastisch verformbare - Drahteinheit 2 über einen Führungsdraht 4.2 geführt ist, der in das Innere einer flexiblen Elektrodenzuleitung 5 führt, die über eine Verbindungsleitung 3 mit der Drahteinheit 2.2 verbunden ist. Im Inneren der Drahteinheit 2 ist ein mit dem Führungsdraht 4.2 verbundener Ballon 6.2 angeordnet, der die Drahteinheit, wenn er aufgeblasen wird, gegen die Gefäßinnenwand drückt. Auch die mit einem inneren Lumen versehene Zuleitung 5 wird dabei von dem Führungsdraht durchquert. Aus der Abbildung ist erkennbar, daß durch die fehlende elektrische Leitung innerhalb des zylindrischen Querschnitts der Drahteinheit 2.2 auch die Führung des Ballons völlig unbehindert möglich ist.

Bei der Ausführungsform nach Fig. 3 ist - im Gegensatz zur Ausführung gemäß Fig. 2, der Führungsdraht 4.3 für den Ballon 6.3 nicht durch das Innere der die Zuleitung bildenden flexiblen Elektrodenzuleitung (Sonde) 5 geführt.

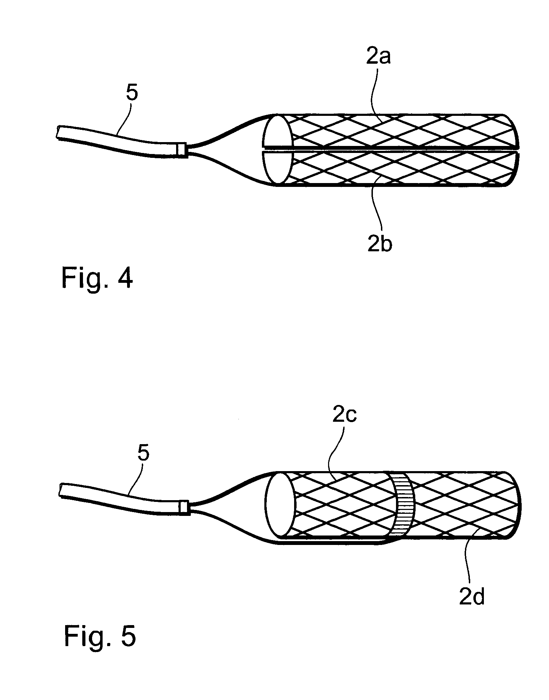

In Fig. 4 und 5 sind bipolare Ausführungsformen einer erfindungsgemäßen Elektrode 1 dargestellt, wobei in der Ausführungsform nach Fig. 4 die Drahteinheit aus zwei in tangentialer Richtung voneinander durch einen Isolierbereich getrennten Teilen 2a, 2b und gemäß Fig. 5 aus in axialer Richtung voneinander isolierten Teilen 2c, 2d besteht. Es ist ersichtlich, dass sich auch nach der Ausführung gemäß der Fig. 4 und 5 kein Teil der Zuleitung im inneren Hohlraum des Drahtgebildes befindet. Bei der Ausführung gemäß Figur 5 gelang die elektrische Verbindung außerhalb des Teils 2c zu dem Teil 2d. Er kann alternativ auch innerhalb des Wandungsbereichs des dann als Geflecht ausgebildeten Teils 2c isoliert geführt sein. Hierbei wäre dann die elektrische Verbindung mit einer isolierenden Ummantelung zu versehen, damit nicht eine leitende Verbindung zu dem Teil 2c hervorgerufen wird.

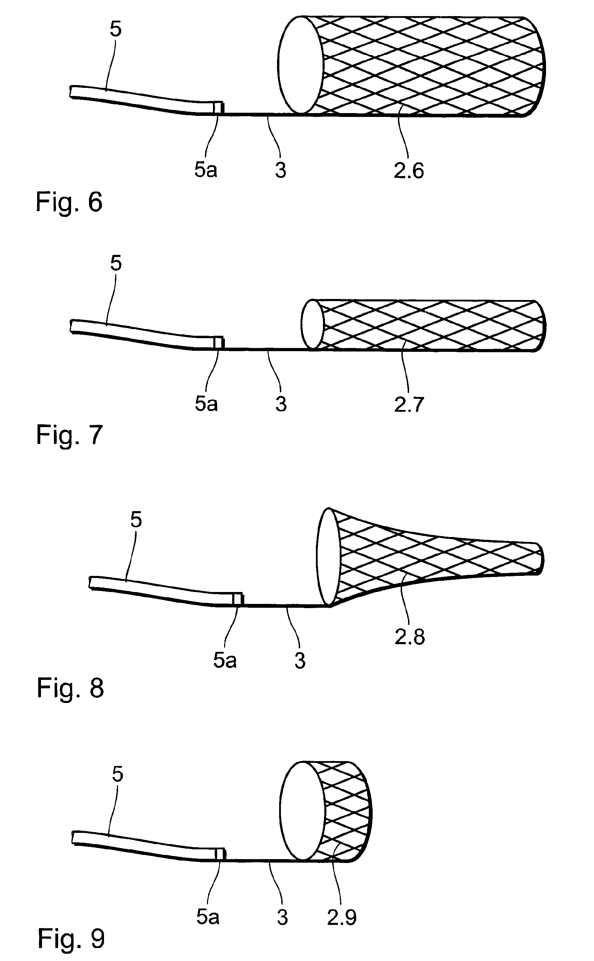

In Fig. 6 und 7 sind zylinderförmige Drahteinheiten 2.6 und 2.7 unterschiedlichen Durchmessers dargestellt.

Fig. 8 zeigt eine Drahteinheit 2.7, welche sich an einem Ende 6 konisch aufweitet.

In Fig. 9 ist eine schmale ringförmige Drahteinheit 2.9 veranschaulicht, welche eine Länge von ca. 5 mm aufweist.

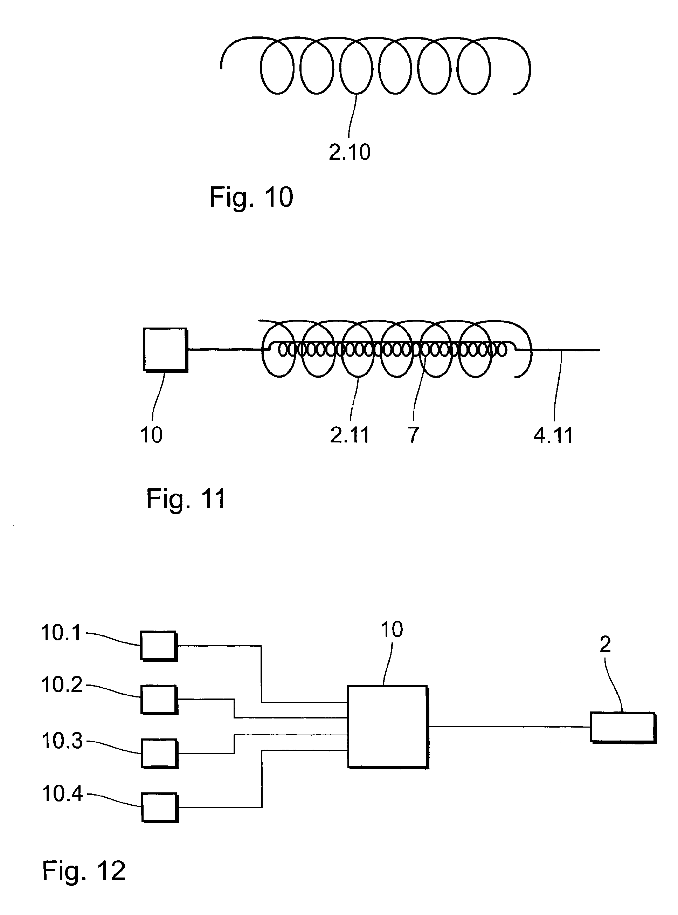

Fig. 10 zeigt ein Ausführungsbeispiel, bei welchem die Drahteinheit 2.10 die Form einer Zylinderspule aufweist, so daß eine induktive Aktivierung von außen ermöglicht wird.

Bei der Variante nach Fig. 11 ist auf dem Führungsdraht 4 im Bereich des Inneren der spulenförmigen Drahteinheit 2.11 eine Spule 7 zur Erzeugung einer Induktionsspannung angeordnet. Die Spule 7 wird von einer Steuereinheit 10 gespeist.

Bei der in Fig. 12 dargestellten Ausführungsform ist eine Elektrode der vorbeschriebenen Bauart mit einer Stimulations-Steuereinheit 10 versehen, welche mit Signaldetektoren 10.1 bis 10.4 für verschiedene Eingangssignale versehen ist.
- Stimulation parasympathischer autonomer Nervenfasern zur Senkung der Herzvorhof- und Hauptkammerfrequenz bei tachykarden Herzrhythmusstörungen. Die Implantation der einen Expansionskörper bildende Drahteinheit kann zu diesem Zweck in der arteria/vena jugularis interna oder externa, der oberen Hohlvene, dem proximalen Koronarsinus oder der unteren Hohlvene an der Grenze zum rechten Vorhof erfolgen.
- Stimulation autonomer Nervenfasern zur Verbesserung der Koronararteriendurchblutung. Die Implantation der einen Expansionskörper bildende Drahteinheit kann zu diesem Zweck in der arteria/vena jugularis interna oder externa sowie im Koronarsinus erfolgen.
- Stimulation sympathischer autonomer Nervenfasern zur Behandlung einer arteriellen Hypotonie und Herzpumpenschwäche bei akuter und chronischer Herzinsuffizienz. Die Implantation der einen Expansionskörper bildende Drahteinheit kann zu diesem Zweck in der arteria/vena subclavia, den Pulmonalvenen oder der Aorta erfolgen.
- Stimulation sympathischer autonomer Nervenfasern zur Behandlung einer arteriellen Hypotonie und Bradykardie bei neurokardiogenen Synkopen. Die Implantation der einen Expansionskörper bildende Drahteinheit kann zu diesem Zweck in der arteria/vena subclavia, den Pulmonalvenen oder der Aorta erfolgen.
- Stimulation parasympathischer autonomer Nervenfasern, zur Behandlung tachykarder ventrikulärer Rhythmusstörungen. Die Implantation der einen Expansionskörper bildende Drahteinheit kann zu diesem Zweck im Koronarsinus oder der Pulmonalarterie erfolgen.
- Stimulation parasympatischer Nerven, die die Vorhöfe innervieren zur Verhinderung eines atrialen Remodelling Prozesses. Die Implantation der einen Expansionskörper bildende Drahteinheit kann zu diesem Zweck in der arteria/vena jugularis interna oder externa, der oberen Hohlvene oder der rechten Pulmonalarterie erfolgen.
- Stimulation parasympathischer Nerven, die die Vorhöfe/Ventrikel innervieren Senkung der atrialen/ventrikulären Defibrillationsschwelle. Die Implantation der einen Expansionskörper bildende Drahteinheit kann zu diesem Zweck in der arteria/vena jugularis interna oder externa, der oberen Hohlvene oder der rechten Pulmonalarterie erfolgen.
- Stimulation autonomer parasympathischer Nervenfasern zur Behandlung von zerebralen Krampfleiden (Epilepsien). Die Implantation der einen Expansionskörper bildende Drahteinheit kann zu diesem Zweck in der arteria/vena jugularis interna oder externa erfolgen.
- Stimulation der Karotissinusnerven zur Behandlung von angina pectoris Beschwerden. Die Implantation der einen Expansionskörper bildende Drahteinheit kann zu diesem Zweck in der arteria/vena jugularis interna oder externa erfolgen.
- Stimulation autonomer Nerven, die die Magen-/Darm- und Blasenmotilität regulieren sowie die männliche Erektion steuern. Die Implantation der einen Expansionskörper bildende Drahteinheit kann zu diesem Zweck in der vena cava inferior und ihren Zuflüssen, der aorta abdominalis sowie ihrer Abgänge (z.B. aa. mesentericae) oder den arteriellen und venösen Ilikalgefäßen erfolgen.
- Hochfrequente, unterschwellige elektrische Stimulation des ventrikulären Myokards zur Förderung der Angiogenese nach Herzinfarkten oder myokardialen Durchblutungsstörungen. Die Implantation der einen Expansionskörper bildende Drahteinheit kann hierzu in den Koronararterien oder dem Koronarsinus und seinen Zuflüssen erfolgen.

Die Sensoren 10.1 bis 10.4 stellen dabei beispielhaft Elemente dar, welche den Zustand der Aktivierung für die Stimulations-Steuereinheit 10 entsprechend erfühlen und veranlassen, daß das diese eine entsprechende Steuerspannung bzw. Steuerstrom geeigneter Form, Dauer und gegebenenfalls Frequenz an die nachgeschaltete Drahteinheit abgeben.

## Patentansprüche

1. Elektrode zur intravaskulären Nervenstimulation, Kardioversion bzw.Defibrillation in Form einer Stimulationssonde, die in arteriellen/venösen Gefäßen des Körpers fixierbar ist und über die elektrische oder magnetische Impulse sowie Defibrillations-/Kardioversionsschocks abgebbar sind, welche mit einer Zuleitung versehen ist, dadurch gekennzeichnet, daß eine in axialer Richtung an die Zuleitung anschließende metallische und elektrisch leitfähige, tubuläre, einen Expansionskörper bildende Drahteinheit vorgesehen ist, die in dem entsprechenden Gefäß entfaltbar ist und sich unter Expansion von innen an die Gefäßwand anlegt.

2. Elektrode nach Anspruch 1, dadurch gekennzeichnet, daß als Antriebsmittel für die Expansion im Inneren der plastisch verformbar ausgebildeten Drahteinheit ein pneumatisch oder hydraulisch aufblasbarer Ballonkörper vorgesehen ist.

3. Elektrode nach Anspruch 1, dadurch gekennzeichnet, daß die Drahteinheit federelastisch ausgebildet ist und mit einer Kompressionshülle umgeben ist, wobei die Expansion des Drahtkörpers selbsttätig durch Entfernung einer Kompressionshülle (Selbstexpansion) von der vorgespannt komprimierten Drahteinheit erfolgt.

4. Elektrode nach einem der Ansprüche 1 bis 3, dadurch gekennzeichnet, daß die gesamte Oberfläche der Drahteinheit oder ein oder mehrere elektrisch voneinander isolierte Teile derselben als unipolarer, bipolarer oder multipolarer Stimulationspol ausgebildet sind.

5. Elektrode nach einem der Ansprüche 1 bis 4, dadurch gekennzeichnet, daß die Drahteinheit nach Art einer Zylinderspule ausgebildet ist.

6. Elektrode nach Anspruch 5, dadurch gekennzeichnet, daß die Zylinderspule eine Mehrzahl elektrisch voneinander isolierter Teilspulen umfaßt.

7. Elektrode nach Anspruch 5 oder 6, dadurch gekennzeichnet, daß eine Induktionseinheit vorgesehen ist, mittels der die Elektrode induktiv mit Spannung beaufschlagbar ist.

8. Elektrode nach einem der Ansprüche 1 bis 7, dadurch gekennzeichnet, daß der Durchmesser der Drahteinheit sich in Längsrichtung ändert.

9. Elektrode nach Anspruch 8, dadurch gekennzeichnet, daß zum Einsatz in den proximalen Koronarsinus die Drahteinheit konisch ausgebildet ist.

10. Elektrode nach einem der Ansprüche 1 bis 9, dadurch gekennzeichnet, daß die Oberfläche mit einem Medikament, insbesondere mit einer Substanz zur Vermeidung von Gefäßschädigungen, beschichtet ist.

11. Elektrode nach Anspruch 7, dadurch gekennzeichnet, daß eine Induktionseinheit vorgesehen ist, mittels der die Elektrode induktiv beheizbar ist.

12. Elektrode nach einem der vorangehenden Ansprüche, dadurch gekennzeichnet, daß ein weiterer Teil der Zuleitung, welcher sich in Längsrichtung parallel mindestens zu einem Teil der Drahteinheit erstreckt, als isolierter Bereich innerhalb des zylindrischen durch die Drahteinheit erzeugten Wandungsbereichs oder außerhalb derselben verläuft.

13. Elektrode nach einem der vorangehenden Ansprüche dadurch gekennzeichnet, daß eine Steuereinheit vorgesehen ist, welche Steuersignale für eine oder mehrere der nachfolgenden Anwendungen erzeugt und mit der Drahteinheit elektrisch verbunden ist:
- Stimulation parasympathischer autonomer Nervenfasern zur Senkung der Herzvorhof- und Hauptkammerfrequenz bei tachykarden Herz-rhythmusstörungen, wobei die Implantation der einen Expansionskörper bildende Drahteinheit in der arteria/vena jugularis interna oder externa, der oberen Hohlvene, dem proximalen Koronarsinus oder der unteren Hohlvene an der Grenze zum rechten Vorhof erfolgt;
- Stimulation autonomer Nervenfasern zur Verbesserung der Koronararteriendurchblutung, wobei die Implantation der einen Expansionskörper bildende Drahteinheit kann zu diesem Zweck in der arteria/vena jugularis interna oder externa sowie im Koronarsinus erfolgt;
- Stimulation sympathischer autonomer Nervenfasern zur Behandlung einer arteriellen Hypotonie und Herzpumpenschwäche bei akuter und chronischer Herzinsuffizienz, wobei die Implantation der einen Expansionskörper bildende Drahteinheit kann zu diesem Zweck in der arteria/vena subclavia, den Pulmonalvenen oder der Aorta erfolgt;
- Stimulation sympathischer autonomer Nervenfasern zur Behandlung einer arteriellen Hypotonie und Bradykardie bei neurokardiogenen Synkopen, wobei die Implantation der einen Expansionskörper bildende Drahteinheit kann zu diesem Zweck in der arteria/vena subclavia, den Pulmonalvenen oder der Aorta erfolgt;
- Stimulation parasympathischer autonomer Nervenfasern, zur Behandlung tachykarder ventrikulärer Rhythmusstörungen, wobei die Implantation der einen Expansionskörper bildende Drahteinheit im Koronarsinus oder der Pulmonalarterie erfolgt;
- Stimulation parasympatischer Nerven, die die Vorhöfe innervieren zur Verhinderung eines atrialen Remodelling Prozesses, wobei die Implantation der einen Expansionskörper bildende Drahteinheit in der arteria/vena jugularis interna oder externa, der oberen Hohlvene oder der rechten Pulmonalarterie erfolgt;
- Stimulation parasympathischer Nerven, die die Vorhöfe/Ventrikel innervieren Senkung der atrialen/ventrikulären Defibrillationsschwelle, wobei die Implantation der einen Expansionskörper bildende Drahteinheit in der arteria/vena jugularis interna oder externa, der oberen Hohlvene oder der rechten Pulmonalarterie erfolgt;
- Stimulation autonomer parasympathischer Nervenfasern zur Behandlung von zerebralen Krampfleiden (Epilepsien), wobei die Implantation der einen Expansionskörper bildende Drahteinheit in der arteria/vena jugularis interna oder externa erfolgt;
- Stimulation der Karotissinusnerven zur Behandlung von angina pectoris Beschwerden, wobei die Implantation der einen Expansionskörper bildende Drahteinheit kann zu diesem Zweck in der arteria/vena jugularis interna oder externa erfolgt;
- Stimulation autonomer Nerven, die die Magen-/Darm- und Blasenmotilität regulieren sowie die männliche Erektion steuern, wobei die Implantation der einen Expansionskörper bildende Drahteinheit in der vena cava inferior und ihren Zuflüssen, der aorta abdominalis sowie ihrer Abgänge oder den arteriellen und venösen llikalgefäßen erfolgt;
- Hochfrequente, unterschwellige elektrische Stimulation des ventrikulären Myokards zur Förderung der Angiogenese nach Herzinfarkten oder myokardialen Durchblutungsstörungen, wobei die Implantation der der einen Expansionskörper bildende Drahteinheit in den Koronararterien oder dem Koronarsinus und seinen Zuflüssen erfolgt.
